# EUROPEAN PATENT SPECIFICATION

(11) **EP 1 865 838 B1**
(45) Date of publication and mention of the grant of the patent: **01.02.2012**
(21) Application number: 06728506.4
(22) Date of filing: 23.03.2006
(51) Int. Cl.: A61B 5/0496

(54) **GLAUCOMA SCREENING APPARATUS**
GLAUKOM-SCREENINGGERÄT
APPAREIL DE DEPISTAGE DU GLAUCOME

(30) Priority: 04.04.2005 IT FI20050057
(43) Date of publication of application: 19.12.2007
(73) Proprietor: Costruzioni Strumenti Oftalmici C.S.O. S.r.l., 50010 Scandicci (Firenze) (IT)
(72) Inventor: BAGLINI Claudio, 56010 Migliarino Pisano (Pisa) (IT)
(74) Representative: Bardini, Marco Luigi
(86) International application number: PCT/IT2006/000185
(87) International publication number: WO 2006/106548

(56) References cited:
- WO-A-03/065876
- US-A- 5 539 482
- HASEGAWA S ET AL: "Waveform changes of the first-order multifocal electroretinogram in patients with glaucoma." INVESTIGATIVE OPHTHALMOLOGY & VISUAL SCIENCE. MAY 2000, vol. 41, no. 6, May 2000 (2000-05), pages 1597-1603, XP002387429 ISSN: 0146-0404
- HORN F ET AL: "Quadrant pattern ERG with SLO stimulation in normals and glaucoma patients." GRAEFE'S ARCHIVE FOR CLINICAL AND EXPERIMENTAL OPHTHALMOLOGY = ALBRECHT VON GRAEFES ARCHIV FÜR KLINISCHE UND EXPERIMENTELLE OPHTHALMOLOGIE. AUG 1996, vol. 234 Suppl 1, August 1996 (1996-08), pages S174-S179, XP008065885 ISSN: 0721-832X
- GRAHAM S L ET AL: "Pattern electroretinograms from hemifields in normal subjects and patients with glaucoma." INVESTIGATIVE OPHTHALMOLOGY & VISUAL SCIENCE. AUG 1994, vol. 35, no. 9, August 1994 (1994-08), pages 3347-3356, XP002387430 ISSN: 0146-0404

## Description

### Field of the invention

The present invention relates to the field of glaucoma diagnostics and, in particular, it refers to glaucoma screening using a pattern electroretinogram (PERG).

### Background of the invention

Glaucoma is an eye disease characterized by a strong increase in the ocular bulb tension, caused by hindered endocular fluid drain which may lead to severe visual loss and, eventually, to blindness.

It is very difficult for an ophthalmologist to diagnose glaucoma before visual field alterations or optic nerve anatomy modifications occur. Unfortunately, when those symptoms arise, a considerable quantity of ganglion cells and optic nerve fibers have already been destroyed by the pathology. In fact, scientific literature indicates that the symptoms are associated with a loss of optic nerve fibers in the retina in the range of at least 30-50%. Therefore, it is important for the ophthalmologist to be able to detect retinal ganglion cell abnormalities or loss at an early stage, before severe visual defects occur.

A well-known and currently used early glaucoma detection system is the pattern electroretinogram (PERG). This is a test that has been widely described by scientific literature. As disclosed for example in PCT international patent publication n. WO03/065876, it consists mainly in assessing the retinal ganglion cell response to a so-called "pattern" stimulation, consisting of a sequence of images having horizontal or vertical bars, or black and white checks, the sequence being displayed on a screen with a specific luminance and contrast. The patient observes these visual stimuli which alternate with a predetermined frequency and, simultaneously, the corresponding electroretinogram is recorded through suitable electrodes placed at the conjunctival fornix (of both eyes in case of binocular recording). From the electroretinogram, the so-called retinal biopotential is drawn out. The latter represent a measure of the retinal integrity (density of unaltered ganglion cells) in relation to the possible glaucomatous damage already produced.

The current techniques obtain a PERG generated by stimulating simultaneously the entire retinal surface in order to compare it with predetermined reference values, thus attaining a diagnostic outcome. However, these techniques are not totally reliable, especially with early stage glaucoma, because the comparison with external reference values obtained from previous diagnostic tests depends in part on interindividual variability.

In fact, each individual's peculiarity (refractive defects, age and so on) possibly due to pathologies different from glaucoma, but also simple environmental factor variations differing in each recording, make the comparison not entirely reliable. It is not uncommon in practice that an alleged glaucoma diagnosis turns out to be caused by another pathology (for example, cataract) and, on the contrary, a negative diagnosis turns out to be wrong later on.

US Patent No. 5539482 in the name of James/Maddess gives the possibility of analyzing the retina by dividing it in different predetermined areas. The purpose of this area by area analysis is primarily that of localizing the possible damage more accurately, but the diagnostic outcome is obtained with the same methodology described before, that is, by comparison with statistically normal values obtained following clinical trials on healthy patients. Thus the reliability limits are similar. A further known apparatus according to the preamble of annexed claim 1 is disclosed in HASEGAWA ET AL: "Waveform changes of the first-order multifocal electroretinogram in patients with glaucoma." INVESTIVATIVE OPHTALOMLOGY & VISUAL SCIENCE. MAY 200, vol. 41, no. 6, May 2000 (2000-05)

### Summary of the invention

The object of the present invention is to overcome the above situation by providing apparatus suitable to prevent uncertain measurements connected to the intrinsic variability of the outcome in traditional glaucoma screening clinical practice.

A further object of the present invention is to simplify the PERG recording procedure to make the diagnosis easy to perform, reproducible and fast.

These objects are achieved with the glaucoma screening apparatus according to the present invention, whose essential characteristics are set forth in the first of the annexed claims.

### Brief description of the drawings

The characteristics and advantages of the glaucoma screening apparatus according to the present invention will be apparent from the description of embodiments thereof, given purely by way of exemplificative and no limitative, with reference to the enclosed drawings in which:
- figure 1 shows the correct positioning on a patient's face of electrodes needed to perform a diagnostic test according to the invention;
- figure 2 shows a schematic representation of the hardware of a diagnostic apparatus for the pattern electroretinogram (PERG) according to the invention;
- figure 3 is a schematic representation of an alternative apparatus for the pattern electroretinogram (PERG) according to the invention;
- figure 4 shows an exemplary flowchart of a diagnostic procedure with the appartatus according to the invention.

### Description of the preferred embodiments

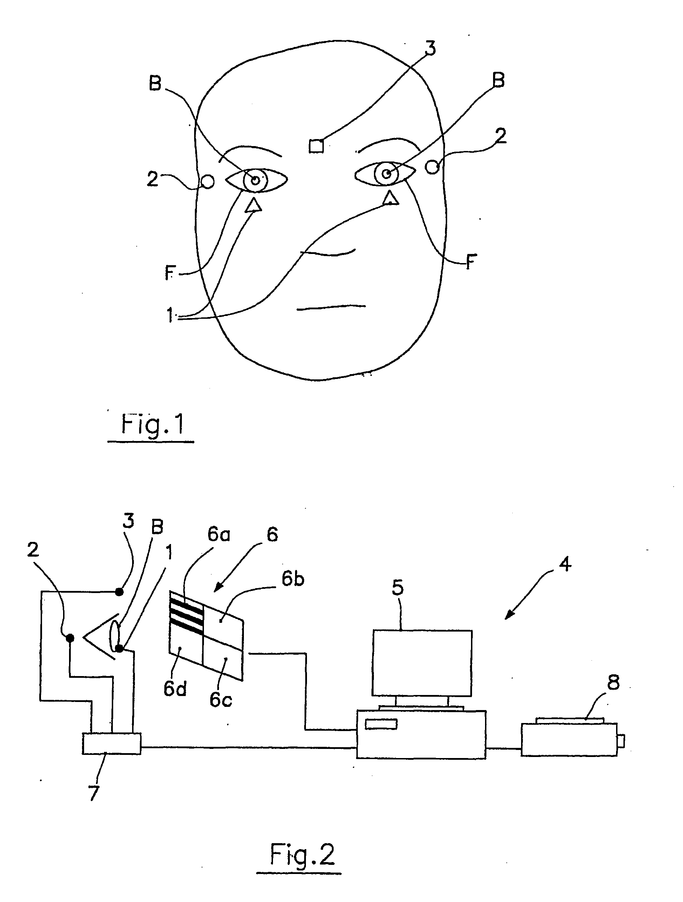

Referring to figure 1, according to the invention an arrangement of electrodes is placed on the face of a patient under examination following traditional PERG technique guidelines. In particular, an exploring active electrode 1 is inserted into the eye in the lower conjunctival fornix F, contacting the ocular bulb B to pick up a bioelectric signal generated by the stimulated retinal ganglion cells. This electrode is preferably of the so-called HK-LOOP type, comprising a thin silver wire coated with a Teflon® sheath on which incisions are made to allow contact of the wire with the bulb.

This electrode type does not interfere with patient's vision, allowing him to blink comfortably, which is not the case, for example, in the commonly used corneal electrodes. Moreover, a better contact impedance, and then a more reliable contact, between the electrode and the patient is obtained in comparison to skin electrodes. Refractive problems caused by corneal electrodes, are avoided. Furthermore, it is possible to record the bioelectric signal with a very high signal/noise ratio, so that the examination time of a test is reduced considerably (the number of samples to be taken is reduced).

After cleansing of the skin, a reference electrode 2 is placed at the temporal outer canthus of each eye, and comprises a silver or silver chloride disk to which a special conductive paste is added to promote contact with the patient's skin. A common electrode 3, of the same type as reference electrode 2, is finally placed at the centre of the patient's forehead.

Referring now to figure 2, the patient, wearing the above mentioned electrodes applied as shown in figure 1, is placed at a distance of about 30 cm from an examination apparatus, indicated in its entirety with 4, with his chin resting on an adjustable rest (not shown).

The apparatus 4 comprises a personal computer 5 on which there is installed a software operating system, suitable to manage simultaneously a pattern stimuli sequence, shown onto screen 6 placed at the patient's eye level, and the resulting PERG recording. In accordance with the provisions of the traditional apparatuses, the screen mean luminance is calibrated at 100 candela/square meter with a contrast between black and white elements of 99%.

The biopotential generated by the retinal ganglion cells is recorded via a differential amplifier 7 connected both to the personal computer 5 and to all the electrodes 1, 2 and 3. Such differential amplifier 7 features a pass band ranging from 1 Hz to 100 Hz, and an amplifying factor ranging from 50,000 and 100,000 V/v. A 12-bit A/D converter (not shown) digitizes the signal, all the above according to usual provisions in the known systems.

As mentioned, the resident software in the personal computer 5, besides providing for the processing of the recorded PERG which is then printed by a printer 8, controls the generation of the pattern stimuli shown on screen 6. However, contrary to the known technique, said stimuli are arranged on screen 6 as an image divided symmetrically by two median lines into four quadrants 6a-6d, whereby in each quadrant a different and independent sequence can be displayed.

In particular, as mentioned, each stimulus comprises horizontal and vertical bars, or black and white checks alternating in each quadrant 6a-6d, with predetermined contrast, spatial and temporal modulation features. In clinical trials the following type of stimuli, found to be particularly suitable for selecting M and Y monocellular ganglion cells, have been especially used: black and white horizontal bars, with a contrast of 99%, a mean luminance of 100 candela/square meter, a spatial frequency of 1.5 cycles/degree and a reversal temporal frequency of 1 Hz for the transient recording, and of 8 Hz for the steady state recording. The stimulation on each quadrant 6a-6d involves a visual angle of about 30° from the centre of screen 6, and subtends a similar visual angle of 30° of radius, centered on the fovea, i.e. the most sensitive part of the retina with the highest ganglion cell concentration. All the above, according to terminology that any PERG expert finds of obvious interpretation.

In this way, four retinal areas, divided into upper and lower retinal hemispheres, in turn divided into temporal and nasal retinal areas, are stimulated independently from each other.

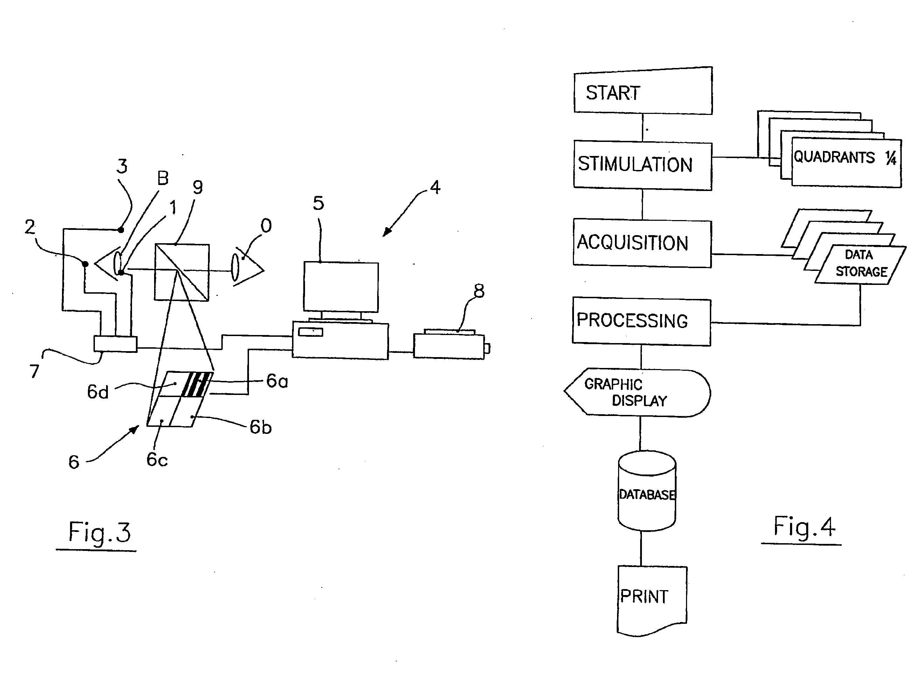

Referring also to figure 4, which is a self-explanatory flow chart, and in greater detail, according to the invention the PERG is recorded to measure the retinal biopotential (possibly also as response density per stimulated area) independently in the four areas which fictitiously divide the retina, according to the different sequence of stimuli on the quadrants 6a-6d of screen 6. Stimulation is performed sequentially on the different quadrants, according to a predetermined pseudo-random progression, so that the patient cannot foresee which quadrant is stimulated next, and, in addition to that, he does not turn his attention away from the centre of screen 6. At the same time, the biopotential linked to the stimulated quadrant and, therefore, to the corresponding relevant retinal area, is acquired.

According to the invention, instead of comparing directly the four measured values obtained with external reference values, they are compared among themselves to ascertain the difference in ganglion cells density between the upper and lower retinal hemispheres. In particular, once the PERG amplitude value is obtained for the lower hemisphere by adding the measured value in the lower nasal quadrant to that in the lower temporal quadrant, the same operation is repeated in the upper hemisphere. Then the two values thus obtained are compared between themselves to obtain their difference in percentage.

In fact, clinical trials have shown that a difference lower than 15% (a single standard deviation) is typical of a healthy subject, and therefore incompatible with the pathology onset, or at least with an already critical development stage. On the contrary, a difference equal to or higher than 30% (two standard deviations) is an indication, with a confidence limit of about 95%, of a decrease in ganglion cells, predictor of glaucoma type condition. With in-between values the patient is monitored through successive examinations in order to ascertain whether the values at issue are physiological or are predictive of the pathology at an early stage.

If the diagnosis is positive, the comparison between complementary nasal and temporal areas of the hemisphere which has returned the lowest absolute value, provides the glaucomatous damage location, given that the retinal damage clearly affects the quadrant with the lowest PERG amplitude.

The table below gives an example of the above described evaluation:

| **Quadrant** | **PERG - amplitude** | **PERG - density per stimulated area** |
|---|---|---|
| Lower temporal | 1.1 µV | 1.222 nV/Deg2 |
| Lower nasal | 1.0 µV | 1.111 nV/Deg2 |
| Upper temporal | 1.2 µV | 1.333 nV/Deg2 |
| Upper nasal | 1.2 µV | 1.333 nV/Deg2 |

Based on the above measurements, the percentage difference between the lower and the upper hemisphere is 12.5% (reduction of 0.3 µV in relation to the total amplitude of 2.4 µV of the upper hemisphere), and consequently the diagnostic response is negative.

The table below shows an example of a positive diagnosis:

| **Quadrant** | **PERG - amplitude** | **PERG - density per stimulated area** |
|---|---|---|
| Lower temporal | 0.7 µV | 0.777 nV/Deg2 |
| Lower nasal | 0.4 µV | 0.444 nV/Deg2 |
| Upper temporal | 1.3 µV | 1.333 nV/Deg2 |
| Upper nasal | 1.2 µV | 1.333 nV/Deg2 |

A percentage difference between the lower and the upper hemisphere is in fact 56% (reduction of 1.1 µV in relation to total amplitude of 2.5 µV of the upper hemisphere).

Therefore, thanks to the invention, by stimulating simultaneously not the entire retinal surface, but the same surface divided into four areas, and assuming that these explored areas give similar responses in a healthy patient and with homogeneous cell density, the difference in the amplitude and phase of the bioelectric response of each examined retinal area indicates whether there is an early alteration of the retinal ganglion cells. This is achieved by focusing and limiting the analysis on the examined patient's intrinsic characteristics without resorting to a comparison with predetermined external values as it is the case with traditional techniques.

Then, the present invention provides a result which is independent from intrinsic factors of variability of the traditional techniques, such as patient's refractive problems, cataract, age, corticosteroid drug use, etc. which can affect the PERG absolute value, thwarting the references obtained from the comparison with normal standards resulting from clinical trials. In the system according to the invention said variability affects both the examined hemispheres in the same way, and, consequently, the coefficient obtained by comparing the two hemispheres is not affected by the above factors. Moreover, the diagnostic outcome is represented by a percentage value which can be clearly, easily and immediately interpreted.

In addition to this, unlike other known diagnostic techniques, according to the invention the stimulated retinal area is very wide (30° centre of fovea) and, thus, involves also the so-called Bjerrum area, which has an extension of about 20-25° in relation to the fovea and whose first pathological modifications in the retinal structure characteristic of glaucomatous condition (for example, Bjerrum scotoma, nasal leap, etc.) are presently detected only at an advanced stage of the pathology (when the nervous fiber loss is already assessed at 30%).

The exemplified apparatus described above allows both patient's retinas to be examined simultaneously. According to a different embodiment diagrammatically shown in figure 3 (in which the parts equal to or corresponding to those already described in the first embodiment are indicated at the same reference numerals), the examination of only one eye at a time is required. On the other hand, this embodiment ensures an even more precise outcome, avoiding contaminating the PERG with possible unreliable recordings.

In particular, the apparatus shown in figure 3 is different from the previous one in that between the patient's eye B and screen 6, placed in this case on a horizontal surface and, like in the previous embodiment, showing an image divided into four symmetrical quadrants 6a-6d, there is a beam splitter of a type per se known, indicated at 9 in figure 3. The beam splitter, arranged so as to interfere with the optic visual pathway, makes it possible to superimpose two different images or observe the same image from two different directions. In this specific case, the beam splitter 9 is inclined by 45°, or another convenient angle, with respect to the said horizontal surface, so that the patient does not look at the stimulation generated by the personal computer 5 directly upon screen 6, but through its reflection upon the splitter.

Thanks to an ophthalmoscope (not shown), the medical practitioner represented in the figure by an eye 0 can observe the patient's retinal fundus during the examination, ensuring that the stimulus is projected exactly onto the centre of fovea. Accordingly, the practitioner is able to detect if the patient stops fixating the stimulus due to, for example, blinking or poor attentive skills in following the same examination, and react immediately, by interrupting and resuming the examination later. The practitioner can also interrupt the recordings temporarily to resume it later, if the patient is in transitory discomfort. The precise projection of the stimulus on the foveal area ensures that the effects of unreliable measurements, caused by the lack of patient's cooperation, are minimized.

In a particularly beneficial embodiment, the beam splitter, the ophthalmoscope and a suitable pattern projection optical system are integrated in a single optical-mechanical system, so that the ophthalmoscope observation field and the pattern projection field superimpose exactly and inseparably. Through this measure, the practitioner knows exactly which retinal area is stimulated, even without seeing the stimulation image reflected by it.

With the embodiments described, even a traditional comparison between the patient's response and the patient's age-related normal data obtained from clinical trials can be obtained, in a more precise and reliable manner, reducing considerably the variability coefficient of the PERG technique. The diagnostic result is truly independent of the operator's ability and the patient's will, as well as of the other previously described variability factors and sources.

In both embodiments, storing the results obtained with a view to a further diagnosis, makes it possible to monitor the progression of the pathology in course of time, and the efficacy of a drug treatment.

Changes and/or modification can be brought to the glaucoma screening apparatus according to the present invention, without departing from the scope of protection of the invention itself as defined by the appended claims.

## Claims

1. A glaucoma screening apparatus (4) for detecting glaucoma in a patient comprising:
- a plurality of electrodes (1,2,3) to be attached to said patient;
- display means (6) for displaying a pattern stimulation to be observed by said patient;
- processing means (5) connected to said electrodes (1,2,3) and said display means (6), adapted to receive signals from said electrodes (1,2,3) in response to said pattern stimulation, and to process said signals to provide an outcome which determines whether the patient suffers from glaucoma;
said processing means (5) being adapted to generate on said display mean (6) a stimulation stimulating independently, on at least one of said patient's eyes, the four retinal areas resulting from the division into upper and lower retinal hemispheres, further divided into temporal and nasal retinal areas, said outcome being obtained by calculating the difference of signals between said upper retinal hemisphere and said lower retinal hemisphere, and then comparing said difference with a predetermined set of values, the apparatus being **characterized, in that** said outcome is obtained by adding the signal from the lower nasal quadrant to the signal of the lower temporal quadrant, then by adding the signal from the upper nasal quadrant to the signal of the upper temporal quadrant, by calculating the difference between the two values thus obtained, by obtaining the percentage value of said difference in relation to the value of the sum in the upper hemisphere, and by comparing said percentage to a predetermined set of values including 15% and 30%, so that if the percentage difference is lower than 15%, the diagnostic outcome is negative, while if the percentage difference is higher than 30%, the diagnostic outcome is positive.

2. The apparatus according to claim 1, in which said display means (6) comprise a screen, said pattern stimulation comprising four independent stimulations generated on respective quadrants (6a-6d) of said screen, obtained by dividing the screen with two median lines, namely a horizontal and a vertical one.

3. The apparatus according to claim 2, in which said stimulations are generated in sequence on said quadrants according to a predetermined pseudo-random succession.

4. The apparatus according to any of the claims from 1 to 3, in which said pattern stimulation comprises black and white horizontal bars with a percentage contrast of 99%, having a mean luminance of 100 candela/square meter, a spatial frequency of 1.5 cycles/degree and a reversal temporal frequency of 1 Hz for the transient recording and 8 Hz for the steady state recording.

5. The apparatus according to any of the claims from 2 to 4, in which said stimulation applied to each of the said quadrants affects a visual angle of about 30° from the centre of said screen, and subtends a similar visual angle of about 30° of radius, centered on the fovea of said patient's eye.

6. The apparatus according to any of the claims from 1 to 5, in which between said patient and said display means there are arranged beam splitter means (9) for said patient to observe said pattern stimulation reflected by said beam splitter means instead of directly by said display means, so that the medical practitioner can observe for a diagnostic evaluation the patient's retinal fundus during the same examination.

7. The apparatus according to claim 6, in which said beam splitter means (9), said display means (6) and ophthalmoscope means to observe the patient's retinal fundus during the examination, are integrated into a single optical-mechanic device, so that the observation field of said ophthalmoscope means and that of the pattern stimulation projection superimpose exactly and indivisibly.

## Patentansprüche

1. Glaukom-Screeninggerät (4) zum Detektieren von Glaukom in einem Patienten beinhaltend:
- eine Vielzahl von Elektroden (1, 2, 3) zum Anbringen an dem Patienten;
- Anzeigeeinrichtungen (6) zum Anzeigen einer Musterstimulation zum Beobacthen von dem Patienten;
- mit den Elektroden (1, 2, 3) und dem Anzeigeeinrichtungen (6) verbundene Verarbeitungseinrichtungen (5), die darauf ausgelegt sind, Signale von den Elektroden (1, 2, 3) in Abhängigkeit von der Musterstimulation zu empfangen und die Signale zu verarbeiten, um ein Resultat bereit zu stellen, das bestimmt, ob der Patient an Glaukom leidet;
wobei die Verarbeitungseinrichtungen (5) ausgelegt sind, um an den Anzeigeeinrichtungen (6) eine Stimulation zu erzeugen, die auf wenigstens einem Auge des Patienten die vier retinalen Bereiche unabhängig stimuliert, die aus der Teilung in obere und untere retinale Hemisphären resultieren, die ferner in temporale und nasale retinale Bereiche unterteilt sind, wobei das Resultat erlangt wird, indem die Differenz von Signalen zwischen der oberen retinalen Hemisphäre und der unteren retinalen Hemisphäre errechnet wird, und dann die Differenz mit einem vorgegebenen Satz von Werten verglichen wird, wobei das Gerät **dadurch gekennzeichnet ist, dass** das Resultat erhalten wird durch Addieren des Signals von dem unteren nasalen Quadranten zu dem Signal des unteren temporalen Quadranten, dann durch Addieren des Signals von dem oberen nasalen Quadranten zu dem Signal des oberen temporalen Quadranten, durch Berechnen der Differenz zwischen den zwei somit erhaltenen Werten, durch Erhalten des Prozentwertes der Differenz in Relation zum Wert der Summe in der oberen Hemisphäre, und durch Vergleichen des Prozentsatzes mit einem vorgegebenen Satz von Werten, die 15% und 30% enthaltend, so dass, wenn die Prozentsatzdifferenz geringer als 15% ist, das diagnostizierte Resultat negativ ist, während, wenn der Prozentsatzdifferenz höher als 30% ist, das diagnostizierte Ergebnis positiv ist.

2. Gerät nach Anspruch 1, bei dem die Anzeigeeinrichtungen (6) einen Schirm enthalten, wobei die Musterstimulation vier unabhängige Stimulationen enthält, die an jeweiligen Quadranten (6a - 6d) des Schirms generiert werden, die durch das Unterteilen des Schirms mit zwei Mittellinien erhalten werden, nämlich einer horizontalen und einer vertikalen.

3. Gerät nach Anspruch 2, bei dem die Stimulationen nacheinander an den Quadranten gemäß einer vorgegebenen pseudozufälligen Abfolge erzeugt werden.

4. Gerät nach einem der Ansprüche 1 bis 3, bei dem die Musterstimulation schwarze und weiße Balken mit einem Kontrastprozentsatz von 99% enthält, mit einer mittleren Helligkeit von 100 Candela/Quadratmeter, einer räumliche Frequenz von 1.5 Zyklen/Grad und einer temporale Wechselfrequenz von 1 Hz für die transiente Aufnahme und 8 Hz für die Dauerzustandsaufnahme.

5. Gerät nach einem der Ansprüche 2 bis 4, bei dem die Stimulation, die auf jeden der Quadranten ausgeübt wird, einen visuellen Winkel von etwa 30° vom Zentrum des Schirms betrifft und einem ähnlichen Blickwinkel von etwa 30° Radius gegenübersteht, zentriert an der Fovea des Auges des Patienten.

6. Gerät nach einem der Ansprüche 1 bis 5, bei dem zwischen dem Patienten und den Anzeigeeinrichtungen Strahlteilereinrichtungen (9) angeordnet sind, damit der Patient die Musterstimulation beobachtet, die von den Strahlteilereinrichtungen reflektiert wird, anstatt direkt von den Anzeigeeinrichtungen, so dass der medizinische Fachmann für eine diagnostische Auswertung den retinalen Hintergrund des Patienten während derselben Untersuchung beobachten kann.

7. Gerät nach Anspruch 6, bei dem die Strahlteilereinrichtungen (9), die Anzeigeeinrichtungen (6) und Augenspiegeleinrichtungen zum Beobachten des retinalen Hintergrundes des Patienten während der Untersuchung in eine einzige optischmechanische Vorrichtung integriert sind, so dass sich das Beobachtungsfeld der Augenspiegeleinrichtungen und jenes der Musterstimulationsprojektion exakt und unteilbar überlagern.

## Revendications

1. Un appareil (4) de dépistage du glaucome pour détecter le glaucome chez un patient comprenant:
- une pluralité d'électrodes (1, 2, 3) destinées à être fixées sur ledit patient;
- des moyens d'affichage (6) pour afficher une stimulation réticulée destinée à être observée par ledit patient;
- des moyens de traitement (5) reliés aux dites électrodes (1, 2, 3) et aux dits moyens d'affichage (6), adaptés pour recevoir des signaux provenant des dites électrodes (1, 2, 3) en réponse à ladite stimulation réticulée, et pour traiter lesdits signaux pour donner des résultats qui déterminent si le patient souffre du glaucome;
lesdits moyens de traitement (5) étant adaptés pour créer sur lesdits moyens d'affichage (6) une stimulation stimulant indépendamment, sur au moins un oeil du dit patient, les quatre zones rétiniennes résultant de la division dans les hémisphères rétiniens supérieur et inférieur divisés en outre en zones rétiniennes temporale et nasale, ledit résultat étant obtenu en calculant la différence de signaux entre ledit hémisphère rétinien supérieur et ledit hémisphère rétinien inférieur, et ensuite en comparant ladite différence avec un ensemble de valeurs prédéterminé, l'appareil étant **caractérisé en ce que** le résultat est obtenu en additionnant le signal provenant du quadrant nasal inférieur et le signal du quadrant temporal inférieur, puis en additionnant le signal provenant du quadrant nasal supérieur et le signal du quadrant temporal supérieur, en calculant la différence entre les deux valeurs ainsi obtenues, en obtenant le pourcentage entre ladite différence et la valeur de la somme dans l'hémisphère supérieur, et en comparant ledit pourcentage à un ensemble de valeurs prédéterminé comprenant 15% et 30%, de sorte que si la différence de pourcentage est inférieure à 15%, le résultat du diagnostic est négatif, tandis que si la différence de pourcentage est supérieure à 30%, le résultat du diagnostic est positif.

2. L'appareil selon la revendication 1, dans lequel lesdits moyens d'affichage (6) comportent un écran, ladite stimulation réticulée comportant quatre stimulations indépendantes créées sur des quadrants respectifs (6a-6d) du dit écran, obtenus en divisant l'écran avec deux lignes médianes, à savoir une horizontale et une verticale.

3. L'appareil selon la revendication 2, dans lequel lesdites stimulations sont créées en séquence sur lesdits quadrants selon une succession pseudo-aléatoire prédéterminée.

4. L'appareil selon l'une quelconque des revendications 1 à 3, dans lequel ladite stimulation réticulée comporte des barres horizontales noires et blanches avec un pourcentage de contraste de 99%, ayant une luminance moyenne de 100 candelas/mètre carré, une fréquence spatiale de 1,5 cycles/degré et une fréquence temporelle d'alternance de 1 Hertz pour l'enregistrement transitoire et de 8 Hertz pour l'enregistrement en état stable.

5. L'appareil selon l'une quelconque des revendications 2 à 4, dans lequel ladite stimulation appliquée à chacun des dits quadrants affecte un angle visuel d'environ 30° à partir du centre du dit écran, et sous-tend un angle visuel similaire d'environ 30° de rayon, centré sur la fovéa de l'oeil du dit patient.

6. L'appareil selon l'une quelconque des revendications 1 à 5, dans lequel entre ledit patient et lesdits moyens d'affichage sont prévus des moyens séparateurs de faisceau (9) pour que ledit patient observe ladite stimulation réticulée réfléchie par lesdits moyens séparateurs de faisceau plutôt que directement sur lesdits moyens d'affichage, de façon que, pour une évaluation du diagnostic, le médecin praticien puisse observer le fond rétinien du patient au cours du même examen.

7. L'appareil selon la revendication 6, dans lequel lesdits moyens séparateurs de faisceau (9), lesdits moyens d'affichage (6) et des moyens ophtalmoscopes pour observer le fond rétinien du patient au cours de l'examen, sont intégrés dans un dispositif unique opto-mécanique, de façon que le champ d'observation des dits moyens ophtalmoscopes et la projection de la stimulation réticulée se superposent de façon exacte et indivisible.
